# EUROPEAN PATENT SPECIFICATION

(11) **EP 1 281 438 B1**
(45) Date of publication and mention of the grant of the patent: **11.11.2009**
(21) Application number: 02078345.2
(22) Date of filing: 08.08.1997
(51) Int. Cl.: B01J 38/68, C07C 51/00

(54) **Catalyst recovery**
Katalysatorrückgewinnung
Récupération d'un catalyseur

(30) Priority: 29.08.1996 GB 9617995; 29.08.1996 GB 9617996; 29.08.1996 GB 9617997; 29.08.1996 GB 9617998; 09.12.1996 US 33272 P; 09.12.1996 US 33273 P; 09.12.1996 US 33275; 09.12.1996 US 33277 P
(43) Date of publication of application: 05.02.2003
(62) Divisional of application: 97935690.4
(73) Proprietor: LA SEDA DE BARCELONA S.A., 08820 El Prat De Llobregat, Barcelona (ES)
(72) Inventor: Jeffery, Ian Charles, Stockton-on-Tees, Cleveland TS20 1LP (GB); Dr.Whiston, Keith, Darlington, Durham, DL3 8EY (GB)
(74) Representative: Presland, Torbjörn

(56) References cited:
- EP-A- 0 181 127
- GB-A- 1 364 123
- GB-A- 1 413 488
- US-A- 3 956 175

## Description

This invention relates to the treatment of a catalyst-containing stream derived from the liquid phase oxidation of aromatic polycarboxylic acid precursors to produce the polycarboxylic acid.

Cobalt or manganese or a combination of cobalt and manganese, e.g. in the form of their acetates, together with a source of bromide ion provide catalysis for the catalytic liquid phase oxidation of polycarboxylic acid precursors such as paraxylene to produce the polycarboxylic acid. e.g. terephthalic acid. The liquid phase oxidation is carried out using a lower monocarboxylic aliphatic acid such as acetic acid as a solvent in which the catalyst system is dissolved.

The polycarboxylic acid produced by the oxidation process is withdrawn from the reactor as a slurry of crystals in mother liquor comprising mainly the aliphatic carboxylic acid together with water and dissolved catalyst components and organics (including the polycarboxylic acid, precursors thereof). Further precipitation of the polycarboxylic acid is usually obtained by means of a crystallisation process before separating the crystals from the mother liquor. The solids-liquid separation may be carried out by means of an integrated filtration and washing system as disclosed in EP-A-502628 and WO-A-93/24440, the entire disclosures of which are incorporated herein by this reference.

After separation of the aromatic acid product from the mother liquor from the slurry, conventional practice is to recycle a major part of the mother liquor and its catalyst metal content to the oxidation reactor and to purge a minor part to avoid undue build-up of primarily organic contaminants within the reaction system. The mother liquor purge is treated to recover said aliphatic carboxylic acid for recycle to the oxidation reaction, leaving a high melting point and viscous residue which contains, *inter alia,* metal and bromine catalyst components and organic acidic materials.

It has long been recognised that efficient utilisation of catalyst and process economics call for the further processing of such residues to allow recovery of catalyst metal for reuse in the catalytic liquid oxidation process. The literature is replete with methods for the recovery of the catalyst metals. One commonly used route for recovery involves contacting the residue with water so as to extract the desired metals. Usually the residue is contacted with water in such a way that the metal catalyst components dissolve while the organic contaminants remain largely undissolved. Following separation of the solution from the undissolved components, the solution is contacted with the alkali metal carbonate or bicarbonate to precipitate the catalyst metals as carbonates or bicarbonates so that they can then be recovered for further treatment, if necessary, and recycled to the oxidation reactor.
Such an approach is disclosed for instance in JP-B-81025195, JP-B-79037598-B, JP-B-71014339 and JP-A-51145486 and requires a solids-liquid separation step in order to separate the solution containing dissolved catalyst metals from the undissolved material.

GB 1364123 outlines an aqueous extraction system for recovering Cobalt & Manganese Carbonates from terephthalic acid plant residue. The residue is contaminated by iron and tar-like by-products and cannot be used as such.

The present invention is concerned with the treatment of catalyst containing residues derived from the production of aromatic polycarboxylic acids and seeks to provide an improved process for the recovery of the catalyst metals.

Accordingly the present invention provides a method for recovering heavy metal catalyst from a reaction purge stream which comprises primarily from 85-95% by wt. acetic acid and from 5-15% by wt. water, said method comprising:
(a) boiling off acetic acid to yield a residual liquor comprising catalyst metals and organics;
(b) concentrating the residual liquor by further evaporation of acetic acid to form an evaporator bottoms product in a fluid state;
(c) mixing the evaporator bottoms product with an aqueous medium at a temperature of from 60°C to 80°C and in the presence of 5% w/w caustic soda such that the pH of the resulting mixture is from 4.7 to 5.3, whereby the catalyst metals and organics in the mixture dissolve;
(d) adding to the mixture obtained from step (c) an amount of sodium carbonate or bicarbonate salt which is effective to raise the pH of the mixture to a value in the range of from 6.5 to 9, and adjusting the temperature to 20-60°C, whereby the catalyst metals precipitate as their corresponding carbonate or bicarbonate; and
(e) recovering the catalyst metals.

By dissolving substantially the whole of the residue, the catalyst metal yield can be increased since catalyst metals occluded, chemically or otherwise, with the organics are taken into solution and can then be precipitated as salts, e.g. carbonates and/or bicarbonates.

Preferably the aqueous medium comprises at least in part, for instance at least 10% by weight (e.g. at least 20%), an organic material-containing mother liquor derived from the hydrogenation of an aqueous solution of the polycarboxylic acid. It is conceivable that a major part of the water employed in the dissolution step may be contributed by the water content of said mother liquor. In contrast with prior catalyst recovery schemes in which water is used to extract the desired metals from the residue, the process of the present invention involves the solubilisation of substantially the whole of the residue before precipitating the catalyst metals, thus making it feasible to use an organics containing aqueous medium for dissolution of the metals and organic content of the residue.

Solubilisation of substantially all of the residue in the aqueous medium may be effected by inclusion of an alkaline agent added to the aqueous medium, for instance prior to and/or in the course of combining the residue with the aqueous medium. The agent may comprise ammonium hydroxide or a metal hydroxide, such as sodium hydroxide. Alternatively, pH may be increased initially by the inclusion of said carbonate and/or bicarbonate reaction product in the aqueous medium either as the sole alkaline agent or in combination with another alkaline agent or agents such as ammonium hydroxide or a metal hydroxide. The inclusion of said carbonate and/or bicarbonate reaction product in the aqueous medium (by addition to the aqueous medium prior to or after contacting the same with the residue) is considered advantageous since the metal recovered in the form of carbonates and/or bicarbonates is of a higher quality, apparently because less oxide contamination occurs compared with use of hydroxide only in the initial solubilisation of the residue. For this reason, the process of the invention may be carried out using said carbonate and/or bicarbonate reaction product as the major or sole alkaline agent in effecting initial solubilisation of the residue.

Typically the alkaline agent is introduced to raise the pH sufficiently, preferably to between 4.5 and 5.5 (more preferably 4.7 to 5.3), to dissolve the residue and partially neutralise the acidic content of the residue (and, where applicable, that of the aqueous medium where the latter contains acidic components as in the case where it is constituted by the mother liquor derived from said hydrogenation reaction). Subsequently the carbonate and/or bicarbonate reaction product is added to raise the pH further to precipitate the catalyst metals and secure a pH compatible with downstream processing of the liquor remaining following separation of the solids. For instance, the pH is conveniently increased to about 6.5 to about 9, preferably about 7 to 8, by addition of said carbonate and/or bicarbonate reaction product. Where the downstream processing includes biological treatment, e.g. anaerobic digestion, of the liquor to remove the COD content thereof, the pH of the liquor obtained following precipitation of the metals may be adjusted to 6.5 to 8, preferably about 7, for compatability with the biological treatment process. Such adjustment may involve the addition of further mother liquor derived from the hydrogenation reaction and/or other acidic component such as a mineral acid (e.g. HCI) and/or alkaline component such as caustic soda, in order to secure a suitable pH for biological treatment.

According to a further feature of the present invention there is provided a process for the treatment of heavy metal catalyst-containing residue from the production of an aromatic polycarboxylic acid, comprising the steps of dissolving substantially all of the residue in an aqueous medium, and precipitating the metal catalyst components by inclusion in the aqueous medium of carbonate and/or bicarbonate ions in such a way that evolution of CO₂ is substantially suppressed when the carbonate and/or bicarbonate ions are added.

In one embodiment of the invention, the addition of the carbonate and/or bicarbonate ions is deferred until the pH of the aqueous medium has been increased by addition of an alkaline agent other than a carbonate or bicarbonate to a level such that evolution of CO₂ is substantially suppressed when the carbonate and/or bicarbonate ions are added.

Alternatively or additionally, the extent of dilution of the residue may be controlled such that CO₂ is suppressed upon addition of the carbonate and/or bicarbonate ions.

CO₂ suppression may also be effected by the application of overpressure during the process.

Suppression of carbon dioxide evolution is advantageous in order to avoid stripping volatiles such as acetic acid from the aqueous medium since the evolved gases/vapours would then need to be treated before disposal. Also, suppression of carbon dioxide evolution avoids operating and/or design problems, e.g. foaming and level control, in dealing with such evolution during the addition of the carbonate/bicarbonate ions.

Following precipitation and separation of the catalyst metal carbonates and/or bicarbonates, the liquor is conveniently subjected to anaerobic treatment or wet oxidation, optionally followed by aerobic treatment.

According to yet another aspect of the present invention there is provided a process for the disposal of the organics content of a heavy metal catalyst-containing residue from the production of an aromatic polycarboxylic acid, comprising biologically digesting the organics after neutralising the acidity of said residue for compatability with the biological treatment, the neutralisation step including dissolving said residue in aqueous medium with the aid of an alkaline agent in such a way as to precipitate the heavy metals as salts thereof, preferably as carbonate and/or bicarbonate salts.

The heavy metals may in this way be separated from the liquor (e.g. for recycle to the oxidation reactor) before the liquor is further processed by biological digestion of its organic content thereby substantially eliminating the heavy metals from the sludge produced by the biological digestion process. Thus, the metals precipitation step serves both to recover catalyst values from the residue while at the same time serving as pre-treatment neutralisation of the residue feed to the biological digestion system.

Typically the process of the present invention involves producing the polycarboxylic acid by the oxidation of a precursor thereof (such as p-xylene in the case of terephthalic acid) in a solvent comprising a lower (C2-C6) aliphatic monocarboxylic acid using an oxidising agent, usually air, oxygen enriched gas or substantially pure oxygen, in the presence of a dissolved catalyst system comprising heavy metals such as cobalt and manganese and bromine ions. The polycarboxylic acid produced is extracted from the oxidation reactor in the form of a slurry of crystals in mother liquor mainly comprising the aliphatic carboxylic acid and, following separation of the crystals from the mother liquor (e.g. using one or more integrated solids-liquid separating and water washing units such as are described in EP-A-502628 and WO-A-93/24440), the mother liquor is divided into two fractions for recycle to the oxidation reactor and for purge respectively. The mother liquor purge is concentrated by removal of the aliphatic carboxylic acid (e.g. by evaporation) and the residue is then contacted with said aqueous medium.

In practice removal of any contaminant metals present in the residue, e.g. iron, copper and chromium, may be effected by suitably adapting the teaching of the prior art, for example see GB Patent Nos. 1413488 and 1319172 following the treatment of the present invention.

The invention will now be described by way of example with reference to the accompanying drawings in which:
Figure 1 is a flow sheet illustrating a process for the production of terephthalic acid;
Figure 2 is a flow sheet illustrating a catalyst recovery system in accordance with the process of the present invention; and
Figure 3 is a schematic view of a scrubber unit for effecting scrubbing of off-gas from a reactor used in the production of terephthalic acid.

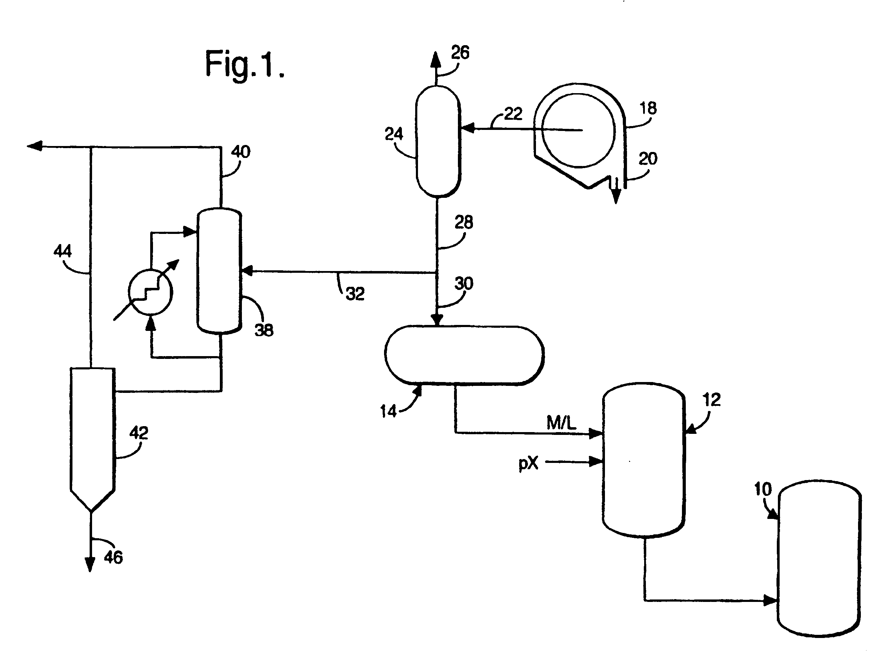

In the process illustrated in Figure 1, terephthalic acid is produced in a reactor 10 by reacting p-xylene (pX) with air (O2) in acetic acid solvent containing some water and a dissolved catalyst system comprising heavy metals, usually cobalt and manganese, and bromine as a promoter. The p-xylene, acetic acid and catalyst may be supplied to the reactor via a feed mix drum 12 in which these components are mixed with recycled mother liquor (M/L) from mother liquor drum 14. The oxygen/air is introduced separately into the reactor 10 via a feed line or lines (not shown). Further details of the reaction are given in our prior European Patent Applications Nos. 498591 and 502628, the entire disclosures of which are incorporated herein by this reference. Typically the reaction is carried out at a temperature of 170-230°C and a pressure of several kg/cm² to 100 kg/cm², eg. 8-30 kg/cm².

The terephthalic acid is withdrawn from the reactor 10 in the form of a slurry of terephthalic acid crystals in mother liquor comprising acetic acid and some water. The slurry is then subjected to crystallisation in one or more crystallisation vessels (not shown) by reducing pressure and temperature so as to precipitate further terephthalic acid. Following the crystallisation process, the slurry is typically at a temperature of the order of 70 to 200°C. The slurry next undergoes an integrated solids-liquid separation process in which the crystals are separated from the mother liquor by filtration and are washed using water or acetic acid as the wash medium. The solids-liquid separation process is carried out in unit 18 under pressure using a filter medium across which a pressure differential is produced to effect displacement of the mother liquor and the wash liquor through the filter cake, comprising terephthalic acid crystals, which develops on the filter medium. The pressure differential may be produced by pressurising the upstream side of the filter medium with a gas or vapour or by hydraulically pressurising the slurry and the wash liqour. The integrated filtration and washing process may be carried out using for example a belt filter as disclosed in European Patent Application No. 502628 under conditions described therein or using a rotary suction filter or a pressure drum filter such as a BHS-Fest drum filter or a centrifuge. In the illustrated embodiment, the filtration and washing process is shown carried out in a rotary filter unit, if desired with countercurrent washing of the filter cake with water. The filter cake comprising terephthalic acid crystals is removed from the unit 18 via line discharge outlet 20 for further processing, e.g. such further processing may comprise preparation for use directly in polyester production (without purification by hydrogenation) or it may comprise purification, for instance by hydrogenation, to reduce the level of impurities in the terephthalic acid followed by subsequent use in the production of polyesters, e.g. as disclosed in our prior International Patent Application No. WO93/24440, the entire disclosure of which is incorporated herein.

The mother liquor filtrate derived from the solids-liquid separation unit 18 via line 22 largely consists of acetic acid (typically 85 - 95% by weight) and water (typically 5 - 15% by weight). The mother liquor also contains soluble organic by-products and intermediates produced in the reaction, reaction catalyst and residual terephthalic acid. Also with this type of filter, the wash liquor often mixes with the mother liquor stream. The recovered mother liquor is fed to a separator 24 in which the liquor is separated from the gas used to provide the pressure difference for the filtration and washing unit 18 (e.g. nitrogen). The gas is recovered via line 26 and the mother liquor via line 28. The mother liquor is split into two fractions, one of which is recycled via line 30 and mother liquor drum 14 back to the reactor and the second of which is purged from the process via line 32 in order to maintain the level of impurities in the system within acceptable limits. The mother liquor recycle fraction is typically in the range 0.7 to 0.99 (e.g. 0.7 to 0.95) and the purge fraction is correspondingly 0.3 to 0.01 (e.g. 0.3 to 0.05).

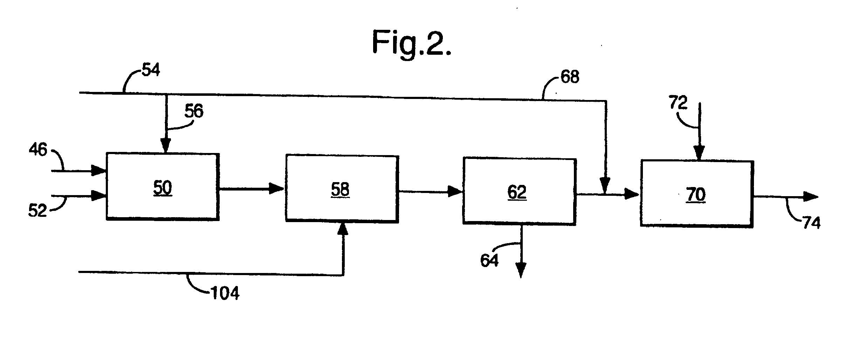

The mother liquor purge is fed via line 32 to a stripper stillpot 38 in which a substantial part of the solvent (acetic acid) is boiled off and fed to an acetic acid recovery process (distillation column) via line 40. The residual liquor is fed to evaporator 42 for concentration. In evaporator 42, further acetic acid is driven off via line 44 for feed to acetic acid recovery in such a way as to leave the evaporator bottoms in a fluid state for supply of the resulting residue to a catalyst recovery waste treatment system (see Figure 2) via line 46. The residue contains, *inter alia*, cobalt, manganese and bromine catalyst components together with acidic organic materials.

Referring to Figure 2, the residue obtained from the evaporator 42 is fed via line 46 to a stirred tank 50 together with a 5% w/w caustic soda solution supplied via line 52 and an aqueous medium supplied via lines 54, 56. Although in Figure 2, the various components are shown as being fed separately to the tank 50, the residue may be slurried up in a portion of the aqueous medium in a slurry receiver upstream of the tank 50. At least part, e.g. at least 10% by weight, of the aqueous medium is advantageously constituted by mother liquor derived from plant for purifying crude terephthalic acid by hydrogenation of an aqueous solution of the crude acid in the presence of a noble metal catalyst such as platinum and/or palladium on an inert, e.g. carbon, support. Suitable plant for purifying crude terephthalic acid is described in EP-A-498591. EP-A-502628 and WO-A-93/24440. As described in these prior patent publications, following hydrogenation the solution is passed through a crystallisation train resulting in a slurry of purified terephthalic acid crystals in aqueous mother liquor and the slurry is filtered and washed. The mother liquor filtrate (primary mother liquor) obtained may be used as the aqueous medium supplied to the tank 50. Alternatively, the primary mother liquor may be subjected to cooling or evaporation to precipitate further, but less pure, terephthalic acid crystals which, following separation from the secondary mother liquor, may be slurried in acetic acid for recycle to the oxidation reactor. The secondary mother liquor so obtained may then be used as the aqueous medium in the catalyst recovery system. If desired, the aqueous medium may comprise both primary and secondary mother liquor. The advantage of using the secondary mother liquor is that Its organic content is reduced compared with the primary mother liquor. Typically the mother liquor supplied to the tank will comprise primarily water but will also contain small amounts of acetic acid, benzoic acid, paratoluic acid terephthalic acid and manganese and cobalt acetates.

In the tank 50, at a temperature of about 60°C to about 80°C, 5% w/w caustic soda is added to raises the pH to for example about 5 and the metals and organics are dissolved. The liquor obtained overflows into a precipitation tank 58 via a baffled outlet to prevent carryover of any solids still undergoing dissolution in tank 50. Sodium carbonate and/or bicarbonate obtained from a scrubber as described below is also supplied to the tank 58 via line 104, the rate of supply being such that the pH is raised to about 6.5 to about 9 leading to precipitation of the catalyst metals, primarily as carbonates and/or bicarbonates thereof. In practice, we have found that significant precipitation of the catalyst metals is not initiated until a pH of about 6 has been attained. Beyond a pH of 6, precipitation increases rapidly and is virtually complete when a pH of 8 is attained. Good metals recovery is achieved at a pH of 7.5.

As mentioned previously, some precipitation of the metals as oxides (especially manganese oxides and/or hydroxides) may also occur especially if caustic soda is used in tank 50. The oxides are considered to be contaminants and consequently it may be preferred to substitute at least part of the caustic soda in tank 50 with sodium carbonate and/or bicarbonate derived from the same source as that supplied via line 104. However, we have found that as long as caustic soda is restricted to raising the pH up to about 5.5 (preferably 4.7 to 5.3), the formation of oxide/hydroxide contaminants is substantially avoided. Sodium carbonate and/or bicarbonate is then used to take the pH up to 6 and beyond. When pH increase is managed in this way, we find that the catalyst metal product recovered is in the form of freely flowing purple powder. If, on the other hand, caustic soda is used to increase pH up to neutral pH, a material is obtained which is very different in appearance, both as a suspension and as a filtered solid, to the carbonate material. In this case, a fine black suspension is obtained which is difficult to filter and forms a brown or black filter cake on filtration which is believed to be attributable to the presence of metal oxides/hydroxides.

The contents of the precipitation tank 58 are passed to solids-liquid separator unit 62 which may, for instance, comprise a clarifier producing a solids-containing underflow and a liquor overflow. The underflow is pumped to a sludge buffer tank (not shown) and subsequently passed to a filter press to produce a relatively dry cake containing the catalyst metal carbonates and/or bicarbonates. The catalyst metals recovered in this way may be recycled via line 64 to the oxidation reactor 10 as their carbonates and/or bicarbonates or, alternatively, before recycle they may be converted to for example acetates by reaction with acetic acid. The unit 62 may alternatively comprise for example a centrifuge or a candle filter unit in which case the filter press may be dispensed with.

The overflow 66 from the clarifier is mixed with additional mother liquor supplied via lines 54, 68 and passes to a final neutralisation tank 70 where, if necessary, acid (e.g. a mineral acid such as HCI) or alkali (e.g. caustic soda) is added via line 72 in order to adjust the pH of the liquor prior to feed to downstream processing plant via line 74. The mother liquor supplied via line 54 typically corresponds to the amount which is to be purged from the purification plant to maintain the levels of impurities within acceptable limits, especially when the mother liquor is to be recycled in the manner disclosed in EP-A-498591, EP-A-502628 and WO-A-93/24440. The purged mother liquor requires treatment before disposal because of its COD and such treatment will usually entail adjustment of its pH.

It will be seen that the process described with reference to Figure 2 allows the purge to be employed as a vehicle for use in recovery of catalyst metals despite the organic content of the mother liquor purge. Rather than pass the entire amount of mother liquor purge to the residue dissolution tank 50, it is preferably divided into two fractions, as indicated by lines 56 and 68, so that the equipment size and cost in these stages can be reduced. Another factor that may influence the amount of mother liquor employed in the dissolution stage (tank 50) is the evolution of CO₂ that occurs in the course of increasing pH in the dissolution stage. If the sodium (or other alkali metal) carbonate and/or bicarbonate is added at low pH levels, for a given amount of the liquor present, the amount of CO₂ that can remain in solution (and hence be available as carbonate ions at the precipitation stage) is reduced compared with addition at higher pH levels.

Consequently to avoid loss of CO₂ from solution on introduction of the sodium carbonate and/or bicarbonate, it may be desirable to secure conditions which suppress CO₂ evolution from the solution.This can be achieved by controlling pH (e.g. a pH of about 5 is suitable) and/or the level of dilution during the dissolution process. Whilst equipment size and cost is a factor which implies minimising the amount of mother liquor used in the dissolution stage, it will be generally desirable to employ sufficient mother liquor consistent with suppressing CO₂ evolution if such evolution is found to be a problem.

The neutralisation carried out in tank 70 will usually involve adjustment of pH within the range 6.5 to 8, preferably 7, for compatability with the downstream processing of the liquor. Such downstream processing may take various forms such as anaerobic treatment (e.g. using the UASB process - upflow anaerobic sludge blanket) followed by aerobic treatment (e.g. activated sludge treatment), or wet oxidation using for example the known ZIMPRO or LOPROX processes.

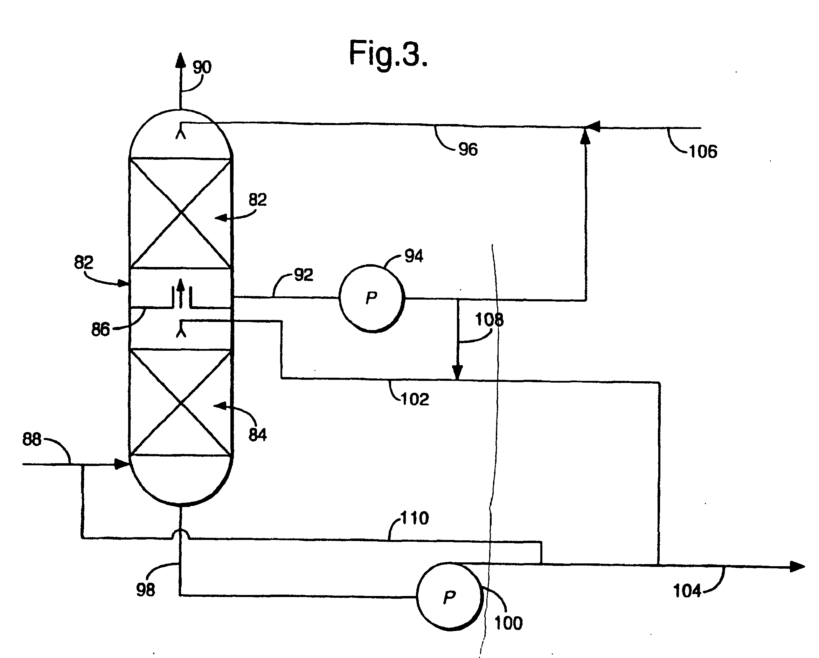

As mentioned in relation to Figure 2, the carbonate used in the treatment of the residue may be derived from a scrubber. Figure 3 illustrates one form of scrubbing unit for use in scrubbing effluent gas from plant for the production of terephthalic acid after treatment of the effluent gas by catalytic oxidation under elevated pressure to convert methyl bromide in the effluent to bromine and/or hydrogen bromide. The effluent gas stream is derived from the overheads condensing system associated with a reactor for the production of terephthalic acid by liquid phase oxidation of p-xylene, for example by means of the process disclosed in our prior EP-A-498591 and/or EP-A-502628, the disclosures of which are incorporated herein by this reference. The effluent gas stream is typically at a pressure of the order of 10 to 16 bara and a temperature of the order of 40°C and typically contains, *inter alia,* volatile organics such as methyl bromide, acetic acid and benzene, together with nitrogen, water vapour, carbon monoxide, carbon dioxide and oxygen.

The gas stream is preheated to a temperature of the order of 250 to 300°C, mixed with a combustion assistant and fed to a catalytic combustion unit. A convenient combustion assistant is methyl acetate which is produced as a by-product in the terephthalic acid production process. Various other combustion assistants may be used instead or in addition, especially those which contain oxygen. The amount of combustion assistant introduced is such that the temperature of the combusted gas stream exiting the catalytic combustion unit is of the order of 400°C or greater. The catalyst employed in the catalytic combustion unit may comprise any suitable oxidation catalyst to secure substantially total conversion of methyl bromide to bromine and HBr while also securing, in combination with the combustion assistant (where needed), substantially total oxidation of other organics such as acetic acid and production of heat to produce the desired exit temperature. Typically the catalyst employed comprises a noble metal catalyst such as platinum and/or palladium supported on an inert support. The support may be ceramic or metallic in the form of a monolith or pellets. Suitable commercial catalysts are available from catalyst manufacturers such as Johnson Matthey, Engelhard and Degussa.

Following catalytic combustion, the treated gas stream typically has a temperature of the order of 400 to 600°C and a pressure only marginally lower than the untreated gas stream, ie about 9.5 to 15.5 bara in the case where the untreated gas stream has a pressure of the order of 10 to 16 bara. The treated gas is then passed through an expander in which the energy content of the gas stream is converted into mechanical power which can be employed appropriately within the terephthalic acid production process, for instance as power input for an air compressor for feeding air under pressure to the oxidation reactor of the production process or for generation of electric power for distribution either within the plant or to other users. At the exit side of the expander, the gas stream temperature is typically of the order of 140 to 200°C (eg about 170°C) and its pressure is near atmospheric, eg about 1.2 bara. The temperature and pressure conditions employed are such that the bromine and HBr derived from methyl bromide in the course of the catalytic combustion remain in the gas phase thereby avoiding any risk of dew point corrosion. In this way, cost penalties otherwise incurred through the use of scrubbing plant upstream of the expander (with consequent reduction in energy available for extraction by means of the expander) or through the use of expensive materials of construction for the expander, are avoided.

Following energy recovery, the gas stream is processed to remove the bromine components so that any discharge to atmosphere is substantially free of such components. Such processing is effected by desuperheating the gas stream and contacting the gas stream with a suitable aqueous scrubbing medium in the scrubbing unit of Figure 3 to remove the Br₂ and HBr so that the bromine content in the discharged gas of less than 4 ppm, with 1 ppm being readily achievable. The scrubbing unit comprises a vessel 80 having two packed sections 82 and 84. The packings employed may be conventional, e.g. Raschig rings, Pall rings etc. A liquid collection tray 86 is located between the two sections 82, 84. The effluent gas (together with water employed to irrigate the pipeline), following treatment to remove HBr, is fed to an inlet 88 at the base of the vessel 80 where the gas and liquid entering the vessel impinge on a plate (not shown) within the vessel base to prevent the gas/liquid mixture impinging on that part of the vessel wall opposite the inlet 88. The gas rises through the vessel, traversing the packed sections 84, 82, and leaves the vessel via outlet 90 which may be a discharge to atmosphere.

The scrubbing liquid employed may be any suitable liquid capable of removing bromine from the effluent gas. The scrubbing liquid is circulated around a loop including inlet line 96, the upper section 82, exit line 92, pump 94 and inlet line 96 so that the liquid flows countercurrent to the direction of gas flow passing up through the vessel 80. A second recirculatory flow of scrubbing liquid is established in the lower part of the vessel 80, again in countercurrent relation to the gas flow, by means of outlet line 98, pump 100 and return line 102. Spent scrubbing liquid is purged from the system via line 104 for supply to the precipitation tank 58 (Figure 2) and make-up liquid is supplied via line 106. The amount of scrubbing liquid pumped through the vessel per unit time will generally be far in excess of that being purged. e.g. a ratio of at least 20:1, e.g. at least 30:1 (typically of the order of 40:1). A purge line 108 interconnects the outlet of pump 94 and line 102 so that scrubbing liquid collecting in the collection tray 86 is passed to the lower recirculatory liquid flow loop. A small amount of the scrubbing liquid is routed to the inlet 88 via line 110, for example from the pump 100, in order to prevent any risk of corrosion in the region of the inlet.

From the foregoing, it will be seen that the bromine containing gas is subjected to a two stage scrubbing treatment allowing the bromine to be substantially completely removed before the gas is discharged from the vessel. The scrubbing liquid is preferably caustic soda, which is converted to sodium carbonate and bicarbonate in the scrubbing vessel as a result of absorption into the hydroxide of carbon dioxide contained in the effluent gas. The sodium (bi)carbonate resulting from the scrubbing process is then used in the recovery of catalyst metals as described above thereby making efficient use of the scrubbing liquor.

### EXAMPLES

### A. Effect of Dissolver pH variations

Samples of catalyst-containing residue derived from a commercial scale plant for the production of terephthalic acid were processed using a laboratory unit comprising a dissolver vessel equipped with an overhead mechanical stirrer, a precipitator vessel also equipped with an overhead mechanical stirrer, and a recovery filter in the form of a reduced pressure Buchner type filter using a vacuum filter cloth. The composition of the plant residue is given in Table 1 below.

**Table 1**

| Typical Unit Residue Feed Composition (all figures ppm wt) | | | |
|---|---|---|---|
| **Component** | **Concentration** | **Component** | **Concentration** |
| 4CBAlc | 3,425 | TMA | 12,292 |
| TA | 39,347 | BPTC | 1,970 |
| 4CBA | 1,115 | p-TOL | 3,258 |
| IPA | 21,236 | Co | 1,400 |
| OPA | 10,482 | Mn | 2,670 |
| BA | 41,408 | Na | 1,070 |
| | | Fe | 33 |

In Table 1, 4-CBAIc is 4-carboxybenzyl alcohol, TA is terephthalic acid, 4CBA is 4-carboxybenzaldehyde, IPA is isophthalic acid, OPA is orthophthalic acid, BA is benzoic acid, TMA is trimellitic acid, BPTC is biphenyltricarboxylic acid, and p-TOL is paratoluic acid.

The plant residue diluted 1:1 with water to render it for pumping and was supplied to the dissolver vessel together with 5% w/w caustic soda to dissolve substantially all of the residue. Typically 4 parts of diluted residue are mixed in the dissolver vessel with 5 parts 2M caustic soda on a weight basis to effect dissolution of the residue. The resulting solution was transferred to the precipitator vessel where it was combined with a feed having a composition corresponding to the caustic scrubber liquor recovered (line 104) from the offgas scrubber system described with reference to Figure 3, namely 3.2% w/w Na₂CO₃/4.8% w/w NaHCO₃. The resulting precipitate was then recovered by filtration. The laboratory unit was operated under different temperature, pH and residence time conditions as set out below in Table 2.

**Table 2**

| Conditions Used in Tests | | | | | |
|---|---|---|---|---|---|
| Run No. | Dissolver Temp | Precipitator Temp | Dissolver pH | Precipitator pH | Dissolver Residence Time (min) |
| 1 | 68.2 | 34.7 | 4.8 | 7.4 | 73.3 |
| 2 | 67.8 | 45.2 | 5.1 | 8 | 84.6 |
| 3 | 78.1 | 49.4 | 6.1 | 8.1 | 49 |
| 4 | 76.9 | 47.7 | 5.9 | 8.2 | 43.2 |
| 5 | 50.6 | 47.7 | 4.5 | 8.1 | 104.2 |
| 6 | 79.1 | 42.1 | 4.5 | 8 | 87.4 |

The filter cake recovered from filtration was dried in air at room temperature and then analysed to determine the amounts of metals and organics present and particle size measurements were also made using a Coulter LS 130 Laser diffraction and PIDS Particle Size Analyser fitted with the Fluid Module, as supplied by Coulter Electronics Limited of Northwell Drive, Luton, Bedfordshire, England, using slurry samples of the recovered catalyst in the process filtrate as the matrix. Metals contents were determined using atomic absorption and organics were quantified using high pressure liquid chromatography. The volume mean particle size is the mean size of the particles in the sample examined on the basis of the volume of material accounted for rather than the number of particles. The 5% quantile mentioned is a measure of the fines content of the sample. It is that particle size (in microns) below which 5% of the total sample lay in the particle size distribution measured, again on a volume rather than number basis. Thus a low 5% quantile figure indicates a higher fines content. It is a more sensitive measure of the small particles in a sample than mean particle size, and may vary widely for samples giving ostensibly similar mean particle size measurements. A lower mean particle size, and more especially a low 5% quantile figure, indicates a finer recovered catalyst material containing a higher proportion of fines which will tend to consist of undesirable metal hydroxide particles. Samples with a high fines content will in general also be more difficult to filter.

The following results (Table 3) were obtained for the different sets of conditions specified above, where "Product" refers to the precipitate recovered by filtration.

**Table 3**

| Analytical Results under Table 2 Conditions | | | | | | | | | |
|---|---|---|---|---|---|---|---|---|---|
| Run No. | Product Co % | Product Mn % | Product Na % | Product Fe ppm | Product Organic Impurities % | Volume Mean Particle Size (micron) | 5% Quantile Volume Basis (micron) | Co Recovery % | Mn Recovery % |
| 1 | 14.6 | 27.3 | 2.34 | 405 | 1.06 | 22.7 | 7.84 | 81.6 | 81 |
| 2 | 14.67 | 28.21 | 1.93 | 490 | 1.23 | 22.7 | 7.84 | 87.2 | 87.9 |
| 3 | 14.75 | 27.78 | 1.96 | 640 | 2.2 | 15.6 | 4.28 | 89.1 | 89.3 |
| 4 | 14.67 | 26.91 | 2.92 | 620 | 4.23 | 15.6 | 4.28 | 89.1 | 89.3 |
| 5 | 12.89 | 25.11 | 2.29 | 630 | 1.46 | 27.7 | 6.45 | 65.4 | 69.2 |
| 6 | 13.86 | 26.2 | 2.49 | 715 | 1.55 | 26.7 | 7.49 | 84.4 | 82.7 |

All figures in Table 3 are weight %. Recovery refers to % metals recovered in the filter cake as a % of those present in residue.

From a comparison of Examples 1 and 2 with Examples 3 and 4. the data in Table 3 reveals that use of caustic soda to adjust the dissolver pH to 6 before the addition of carbonate/bicarbonate produces a material containing significantly higher levels of organic impurities, considerably lower particle size with a higher proportion of fines, and containing more iron. Comparison of Examples 1 and 2 with Examples 5 and 6 reveals that use of less caustic soda to raise the dissolver pH to 4.5 produces a material of equivalent particle size and organics content but the iron content is still higher.

### B. Comparison of Dissolution/Precipitation Regimes

The procedure described in Example A above was carried using the same residue composition using, in Run 1, 5% w/w NaOH in the dissolver vessel and 3.2% w/w Na₂CO₃/4.8% w/w NaHCO₃ (representative of the recovered scrubber liquor composition) in the precipitator vessel as in Example A and, in Run 2, using the 3.2% w/w Na₂CO₃/4.8% w/w NaHCO₃ composition in both vessels. The conditions employed in each Run are given in Table 4. Tables 5 and 6 respectively give the analytical results obtained from Runs 1 and 2 and the metals mass balance.

**Table 4**

| Dissolver/Precipitator Conditions | | | | | |
|---|---|---|---|---|---|
| Run No. | Dissolver Temp | Precipitator Temp | Dissolver pH | Precipitator pH | Dissolver Residence Time min. |
| 1 | 69.58 | 48.88 | 5.05 | 8.05 | 75 |
| 2 | 69.73 | 46.6 | 5.06 | 8 | 71 |

**Table 5**

| Analytical Results under Table 4 Conditions | | | | | | | |
|---|---|---|---|---|---|---|---|
| Run No. | Product Co % | Product Mn % | Product Na % | Product Fe ppm | Product Organic Impurities % | Volume Mean Particle Size (micron) | 5% Quantile Volume Basis (micron) |
| 1 | 14.7 | 25.5 | 2.2 | 1.020 | 0.67 | 16.47 | 4.28 |
| 2 | 13.1 | 25 | 3.1 | 930 | 1.33 | 18.9 | 4.28 |

All percentages in Table 5 are on a weight % basis.

**Table 6**

| Metals Mass Balance | | | |
|---|---|---|---|
| Run No | Mn | Co | Fe |
| 1 | 94.8 | 85.3 | 76.2 |
| 2 | 74.9 | 74.9 | 45 |

In Table 6, the Mn, Co and Fe metals recovery is expressed as a percentage of the metals fed as residue to the dissolver relative to the metals present in the recovered precipitate.

From the results in Tables 5 and 6, it will be seen that the organic acid content of the product is higher for the case using carbonate alone in the dissolution and precipitation steps, and that the metals recovery is also lower in this case. The iron content of the two products is similar although the mass balance suggests that more is purged in the filtrate using carbonate alone. However, less significance is attached to this than to the Co/Mn mass balance information which is more accurate because of the higher concentrations involved.

### C. Comparison of Aqueous Medium

In Example A above, the residue sample was dissolved up using demineralised water in the dissolver vessel. Experimental runs, using the same batch of residue sample, were carried out using:
Run 1 - demineralised water; and
Run 2 - a sample of an aqueous mother liquor (PPML) typically derived as a purge from the hydrogenation stage of a commercially operating terephthalic acid production plant. In both runs, the amount of water added (either as demineralised water or in the form of aqueous mother liquor) were substantially the same.

Both Runs were carried out in accordance with Example A in the laboratory unit using caustic soda in the disssolver vessel and sodium carbonate/bicarbonate in the precipitation vessel (see Table 7 for the conditions used in the laboratory unit).

**Table 7**

| Laboratory unit conditions | | | | |
|---|---|---|---|---|
| Dissolver pH | Dissolver Temp °C | Dissolver Residence Time (minutes) | Precipitator pH | Precipitator Temp°C |
| 5 | 70 | 90 | 7.5 | 60 |

The results of HPLC (high pressure liquid chromatography) analysis showing the organic contents of the recovered precipitate are given in Table 8.

**Table 8**

| Organic Analysis of Recovered Precipitate (all figures ppm wt) | | | | | |
|---|---|---|---|---|---|
| | **4CBAlc** | **TA** | **4CBA** | **IPA** | **OPA** |
| Diluent Water | 155 | 2,417 | 32 | 768 | 309 |
| Diluent PPML | 147 | 1,156 | N/D | 391 | 184 |
| | **BA** | **TMA** | **BPTC** | **p-TOL** | **Total Acids** |
| Diluent Water | 1,633 | 434 | 83 | 126 | 0.73 |
| Diluent PPML | 866 | 246 | 46 | 162 | 0.53 |

All figures in Table 8 are ppm except the total organic acids content which is expressed in wt %.

Given the low levels of organics present, the differences in organic contamination present in the recovered precipitate from each run are not considered to be significant, i.e. for all practical purposes, the use of the aqueous mother liquor from the hydrogenation process is not considered to have a material affect on the quality of the recovered precipitate.

### D. Effect of precipitator temperature

The procedure of Example A was carried out, using the same residue and with water as the diluent, in order to illustrate the effect of temperature on the precipitation stage, particularly in terms of the amount of iron contaminant present in the Co/Mn product recovered. The results obtained are given in Table 9 below.

**Table 9**

| Effect of temperature in precipitation stage | | | | |
|---|---|---|---|---|
| Dissolver Temp °C | Dissolver pH | Precipitator Temp °C | Precipitator pH | Product Iron ppm |
| 70 | 5 | 42 | 7.1 | 850 |
| 70 | 5 | 58 | 7.5 | 945 |
| 70 | 5 | 77 | 7.1 | 3.070 |

From Table 9, it will be seen that the temperature at which precipitation is carried out has a marked affect on the amount of iron contaminating the recovered catalyst product. For this reason, the precipitation stage is operated at a temperature in the range of 20 to 60°C. The temperature of the precipitation stage may be controlled by the temperature of the alkaline agent (e.g. scrubber liquor) introduced into the precipitation stage. If desired, the precipitation vessel may be cooled during the precipitation process to maintain a temperature consistent with reduced iron recovery in the recovered catalyst product.

## Claims

1. A method for recovering heavy metal catalyst from a catalyst containing residue derived from the production of aromatic polycarboxylic acids which comprises primarily from 85-95% by weight acetic acid and from 5-15% by weight water, said method comprising:
(a) boiling off acetic acid to yield a residual liquor comprising catalyst metals and organics;
(b) concentrating the residual liquor by further evaporation of acetic acid to form an evaporator bottoms product in a fluid state;
(c) mixing the evaporator bottoms product with an aqueous medium at a temperature of from 60°C to 80°C and in the presence of 5% w/w caustic soda such that the pH of the resulting mixture is from 4.7 to 5.3, whereby the catalyst metals and organics in the mixture dissolve;
(d) adding to the mixture obtained from step (c) an amount of sodium carbonate or bicarbonate salt which is effective to raise the pH of the mixture to a value in the range of from 6.5 to 9, and adjusting the temperature to 20-60°C, whereby the catalyst metals precipitate as their corresponding carbonate or bicarbonate; and
(e) recovering the catalyst metals.

## Patentansprüche

1. Verfahren zum Rückgewinnen eines Schwermetallkatalysators von einem einen Katalysator enthaltenden Rückstand, der aus der Produktion aromatischer Polycarbonsäuren abgeleitet ist, der primär 85 bis 95 Gewichtsprozent Essigsäure und 5 bis 15 Gewichtsprozent Wasser umfasst, wobei das Verfahren umfasst:
(a) Abkochen von Essigsäure, um eine Restflüssigkeit zu erhalten, die Katalysatormetalle und organische Substanzen enthält;
(b) Konzentrieren der Restflüssigkeit durch weiteres Verdampfen von Essigsäure zur Bildung eines Verdampferbodenprodukts in fluidem Zustand;
(c) Mischen des Verdampferbodenprodukts mit einem wässerigen Medium bei einer Temperatur von 60°C bis 80°C und in Gegenwart von 5% w/w Ätznatron, so dass der pH-Wert der erhaltenen Mischung von 4,7 bis 5,3 reicht, wodurch sich die Katalysatormetalle und organischen Substanzen in der Mischung auflösen;
(d) Zugeben einer Menge an Natriumcarbonatsalz oder Natriumbicarbonatsalz zu der Mischung, das in Schritt (c) erhalten wurde, die effektiv ist, um den pH-Wert der Mischung auf einen Wert im Bereich von 6,5 bis 9 zu erhöhen, und Einstellen der Temperatur auf 20 bis 60°C, wodurch die Katalysatormetalle als ihr entsprechendes Carbonat oder Bicarbonat ausfallen; und
(e) Zurückgewinnen der Katalysatormetalle.

## Revendications

1. Procédé de récupération d'un catalyseur à base de métal lourd à partir d'un catalyseur contenant un résidu dérivé de la production d'acides polycarboxyliques aromatiques comprenant principalement de 85 à 95 % en poids d'acide acétique et de 5 à 15 % en poids d'eau, ledit procédé comprenant :
(a) l'élimination par ébullition de l'acide acétique pour donner une liqueur résiduelle comprenant des métaux catalyseurs et des composés organiques ;
(b) la concentration de la liqueur résiduelle par une évaporation supplémentaire de l'acide acétique pour former un produit au fond de l'évaporateur sous forme liquide ;
(c) le mélange du produit au fond de l'évaporateur avec un milieu aqueux à une température de 60 °C à 80 °C et en présence de 5 % m/m de soude caustique de sorte que le pH du mélange résultant soit de 4,7 à 5,3, les métaux catalyseurs et les composés organiques dans le mélange se dissolvant ;
(d) l'ajout au mélange obtenu dans l'étape (c) d'une quantité de sel de carbonate ou de bicarbonate de sodium qui est efficace pour augmenter le pH du mélange à une valeur comprise dans la plage allant de 6,5 à 9, et l'ajustement de la température à 20 à 60 °C, les métaux catalyseurs précipitant en tant que leur carbonate ou bicarbonate correspondant ; et
(e) la récupération des métaux catalyseurs.
